# EUROPEAN PATENT APPLICATION

(11) **EP 3 889 969 A1**
(43) Date of publication of application: **06.10.2021**
(21) Application number: 20167653.3
(22) Date of filing: 02.04.2020
(51) Int. Cl.: G16H 40/20, G16H 40/63, G06Q 10/06

(54) **MEDICAL IMAGING SYSTEM**

(71) Applicant: Koninklijke Philips N.V., 5656 AG Eindhoven (NL)
(72) Inventor: HELLE, Michael Günter, 5656 AE Eindhoven (NL); AMTHOR, Thomas, 5656 AE Eindhoven (NL); VUPPALA, Sunil Kumar, 5656 AE Eindhoven (NL); WEISS, Steffen, 5656 AE Eindhoven (NL); SISODIA, Rajendra Singh, 5656 AE Eindhoven (NL); JOHNSON, Mark, 5656 AE Eindhoven (NL); VOGMEIER, Gereon, 5656 AE Eindhoven (NL)
(74) Representative: Philips Intellectual Property & Standards

(57) **Abstract**

The present disclosure relates to a method for operating a medical imaging system (100), the method carried out by use of a data processing unit (120), the method comprising:
- obtaining (S1), by a computational prediction model, time-invariant patient information,
- generating (S2), by the computational prediction model, a patient profile parametrized based on at least the obtained time-invariant patient information,
- obtaining (S3), by the computational prediction model, at least one current clinical workflow parameter, and
- providing (S4), by the computational prediction model, a prediction comprising at least a patient-specific operation workflow based on at least correlating the generated patient profile with the obtained current clinical workflow parameter, the predicted patient-specific operation workflow to be used for operating the medical imaging system (100).

## Description

### FIELD OF THE INVENTION

The present disclosure relates to medical imaging. In particular, the present disclosure relates to a medical imaging system, a method for operating a medical imaging system, a method for training a computational model at least partly adapted for operating a medical imaging device, a computer program element, and a computer-readable medium.

### BACKGROUND OF THE INVENTION

It may be desired for medical imaging facilities to have a high patient throughput. At the same time, patient engagement and experience are becoming more important and may be even part of reimbursement in selected markets. Moreover, medical imaging is to become more and more autonomous with less operator-dependent actions and more automated workflow steps.

Decreased patient throughput can be a consequence of multiple different issues within the medical imaging workflow. For example, delayed patient show-up, unexpected patient behavior, e.g. due to lack of information presented to the patient, patient's anxiety etc., patients not able to follow instructions, such as instructions regarding e.g. breath holds, lying still etc. Moreover, image degradation due to artifacts and repeated measurements or even revisits of patients may be caused by improper patient information, so that changes of the medical imaging workflow or imaging protocol adaptions are to be made on-the-fly by, for example, an operator of the medical imaging system.

### SUMMARY OF THE INVENTION

There is, therefore, a need to improve a medical imaging system in terms of optimizing a medical imaging workflow. The object of the present invention is solved by the subject-matter of the appended independent claims, wherein further embodiments are incorporated in the dependent claims.

According to a first aspect, there is provided a method for operating a medical imaging system, the method carried out by use of a data processing unit, the method comprising:
- obtaining, by a computational prediction model, time-invariant patient information;
- generating, by the computational prediction model, a patient profile parametrized based on at least the obtained time-invariant patient information;
- obtaining, by the computational prediction model, at least one current clinical workflow parameter, and
- providing, by the computational prediction model, a prediction comprising at least a patient-specific operation workflow based on at least correlating the generated patient profile with the obtained current clinical workflow parameter, the predicted patient-specific operation workflow to be used for operating the medical imaging system.

In other words, the predicted patient-specific operation workflow may be applied to the medical imaging system in a real-time manner to operate the medical imaging system based on that workflow.

The method may be computer-implemented and may at least partly be carried out by use of the data processing unit, which may be part of the medical imaging system itself or an interrelated system or device. The data processing unit may be functionally coupled to one or more input data interfaces, one or more output data interfaces, one or more data communication interfaces, etc. The method may be implemented in one or more software modules, and/or may be implemented in suitable hardware.

The medical imaging system may further comprise one or more of a Magnetic Resonance Imaging (MRI) device, a computed tomography (CT) device, an X-ray imaging device, or a similar device adapted to acquire medical images, a imaging controller, a database, a data interface to a clinical patient information system, a communication interface, etc.

The computational prediction model may be understood as a model that uses, for example, a functional relationship established by e.g. a regression method or the like to provide a forecast of a dependent variable. It may also be understood as a simulation that outputs a system behavior in response to input data. The prediction model may also comprise at least one neural network, where e.g. dynamic and physical connections of the medical image system are represented by a network structure. There, it is assumed that the system output is a sum of several parts composed of input variables. The model may be compiled based on one or more input data, and may be adapted to be trained and/or adapted by suitable machine learning algorithms.

An effect of this method is that the overall workflow for the medical imaging can be optimized in an at least semi-automated manner, where a computational prediction model, predicts, e.g. calculates, by use of one or more of an algorithm, a neural network, etc., from fed input data, the overall workflow or at least one or more parts of it. The overall workflow may be adapted to a large extent to the specific patient, especially to the patient's behavior. For example, a schedule for examination, a scan protocol based on which the medical imaging system may be operated, a level of staff engagement, etc. may be adapted to the patient's individual needs. This may increase the throughput of a medical imaging facility, as the scan protocol including image acquisition sequences, or the like, may be dynamically adapted and/or optimized. It may also improve the patient engagement and experience, as the workflow may also be adapted to the patient's individual needs in terms of personal support by human staff or by human-machine communication, etc. Further, the method may allow for deciding whether an autonomous imaging process can be used at all or to which extent for the specific patient.

In an embodiment, the method may further comprise:
- determining, by the computational prediction model, a patient-specific risk level assigned to a specific one of a selection of operational modes of the medical imaging system, and
- operating the medical imaging system based on the specific one of the selection of operational modes that is selected depending from the determined risk level.

For example, based on the profiling information and an individual risk analysis calculation for each patient with a personal risk level or factor for an autonomous imaging procedure may be determined, e.g. calculated. Depending on the determination result, the decision for fully-autonomous, semi-autonomous and fully staff supported imaging may be approved and, optionally, documented.

According to an embodiment, the operational mode may be selected depending on whether the determined patient-specific risk level matches an assigned threshold value or an assigned threshold range.

For example, there may be a first threshold value or range assigned to a fully-autonomous operation mode, a second threshold or value or range assigned to a semi-autonomous operation mode, and third threshold value or range assigned to a fully-staff supported. If the risk level matches, for example, the third threshold, semi-autonomous or fully-autonomous operation of the medical imaging system may be prevented by e.g. the data processing unit. Further, if the risk level matches one of the first and second threshold, the level of staff engagement may be decreased by output of respective instructions presented to the staff. Thus, autonomous imaging may be facilitated.

In an embodiment, the selection of operational modes may comprise a fully-autonomous operation mode, a semi-autonomous operation mode, and a fully-staff supported operation mode.

For example, the different operation modes may differ with regard to their degree of automation.

According to an embodiment, the time-invariant patient information may comprise one or more of at least one psychological patient parameter, at least one physiological patient parameter, a patient health status, and historical patient-specific clinical workflow data.

For example, the psychological patient parameter may comprise one or more of or may be indicative for one or more of a certain behavioral pattern, a proneness to anxiety, a risk for unexpected claustrophobia etc., a type of biorhythm, such as "early bird" vs. "night owl", a character type, such as "fighter", which may be referred to as a first character type, "academic", which may be referred to as a second character type, and "social", which may be referred to as a third character type, etc. Thereby, the first character type may indicate a patient preferring fast and efficient action and responding best to instructions delivered in a short and compact manner. Specifically, a suitable written communication style may be a set of bullet points and verbal style a series of orders which just have to be followed. The second character type may, for example, indicate a patient having a craving to understand why they are being asked to follow an instruction. As such a suitable written or verbal communication style may be an explanation of why the instruction is given attached to the instruction itself. The third character type may, for example, indicate a patient having a craving to feel connected to other patients in a similar situation. As such a suitable written or verbal communication style will be an illustration of how somebody similar to specific patient has already undergone the procedure before the instruction is given. One or more of these parameters may be obtained from a questionnaire, which may be provided in paper form and/or electronic form, measurements made to the patient, a behavior analysis based on images, for which images of the patient (shortly) before examination can be taken, either remotely or on site, and evaluated, etc. Thus, the operation of the medical imaging device may be adapted to psychological parameters of the specific patient. For example, one or more of the examination schedule, the human (patient)-machine communication, the scan protocol, the staff engagement, etc. may be, optionally dynamically, adapted to the psychological parameters of the specific patient.

Optionally, the physiological patient parameter may comprise one or more of heart rate, a blood pressure, breathing pattern, ability to hold breath. One or more of these parameters may be obtained from one or more of a questionnaire, which may be provided remotely and/or on site in paper form and/or electronic form, or from measurements made to the patient on site. For example, these data may be measured by a mobile device or other wearable devices (e.g. health band) that comprises, for example, one or more sensors to detect a heart rate, stress level, etc. The measured data may be provided to the medical image system and/or the medical imaging facility via a communication network, or the like. The day, may also be fetched from the data store location (device cloud data) using e-consent. Additionally or alternatively, the data may be measured on-site, which means within the environment of the medical imaging system. Thus, the operation of the medical imaging device may be adapted to physiological parameters of the specific patient. For example, one or more of the human (patient)-machine communication, the imaging schedule, the scan protocol, the level of staff engagement, etc. may be adapted to the physiological parameters of the specific patient.

In an embodiment, the time-invariant patient information may at least be partly remotely obtained in an electronic manner from a computer application to be operated by the specific patient.

Optionally, the computer application can be stored and/or processed on a mobile device, such as a smartphone etc., a personal computer, a cloud computing environment, or the like. The computer application may use one or more of a questionnaire, sensor data processing, etc. to reveal the time-invariant patient information from the specific patient. The computer application may also be adapted to provide the time-invariant patient information electronically to the medical imaging system or a related system, such as a clinical patient information system. Further, the computer application may be adapted to provide a notification to the patient to remind the patient to an upcoming examination. The computer application may also be adapted to provide a user prompt to the patient along with or triggered by the notification. Thus, these data can at least partly be obtained prior to the examination on site at the medical imaging system, without requiring the patient to be on site.

Additionally or alternatively, the time-invariant patient information may in part or completely obtained from e.g. insurance information, from electronic patient records, or the like. These data may comprise, for example, a presence and/or type of an implant, compatibility with imaging modalities etc.

According to an embodiment, the predicted patient-specific operation workflow may comprise one or more of:
- a schedule for examining the specific patient,
- a scan protocol based on which imaging for examining the specific patient is controlled, the scan protocol comprising at least an image acquisition parameter,
- a level of staff engagement for examining the specific patient, the level of staff engagement affecting a level of an autonomous operating mode of the medical imaging system,
- a guidance for staff for examining the specific patient, the guidance comprising one or more of a visualization output, a traffic light system, and an audio output, to be presented to the staff, and
- a type of human-machine communication between the medical imaging system and the specific patient, the type of human-machine communication adapted to the patient profile.

Optionally, predicting the schedule for examining the specific patient may comprise predicting a suitable or optimized time slot for examination.

For example, from the patient profile, an optimal time slot can be advised, e.g. morning sessions for cooperative and not impaired patients and early bird-type patients that present a low probability to delay the workflow while anxious patients may be scanned in later time slots of day as extra time is required to calm them down or even give drugs. Because performance of staff members typically varies over their working time, mostly declining towards the end their shift, exams with difficult patients or scans may be scheduled early on but not first in working shifts. Thus, scheduling may be adapted based or dependent on the predicted patient-specific operation workflow.

Optionally, the scan protocol may be selected from a scan protocol pool. The scan protocol pool may comprise a number of scan protocols previously used for examination of patients having an at least similar patient profile compared with the specific patient. Further, the scan protocol may be adapted, predicted etc. by computationally analyzing an imaging log file, such as a MRI log file etc. For example, parameters such as timing, order, polarity and repetition frequency of RF pulse, applied magnetic field gradients, or the like may be derived from such imaging log file and may be used to predict, adapt etc. the scan protocol.

For example, MRI protocols are the combination of various MRI sequences which can optimally asses a particular region of the body. There is a protocol pool to choose for a particular type of scan. Extracting information from the MRI log file may be used to identify the correct combination. Spin echo sequence can have an adapted field of view, number of slices and sequence of operations with 90 and 180 degree RF pulse, which are captured from the log file. There may be different possibilities to generate the sequence of certain contrast even with pre-defined protocols. The log files may be extracted to identify timing, order, polarity and repetition frequency of RF pulse and applied magnetic field gradients. The extracted log information from similar patients of one particular scan can identify the previous workflow issues and customization of the sequence of operations there by protocols.

Optionally, the level of staff engagement for examining the specific patient may vary between 0 and 1, where 0 means no staff engagement or fully-autonomous operating mode, respectively, and 1 means fully staff supported. Staff engagement level values in between mean a semi-autonomous operating mode.

Optionally, the level of staff engagement may comprise dedicating staff for specific examinations or parts of it, based on one or more of the individual experience or expert level, availability, medical or psychological quality, a type of interaction with patients, etc.

For example, a patient that need complex examination, e.g. cardiac MRI, or patients in a critical current state, such as having a high heart rate, high blood pressure, being nervous, etc., may require specifically experienced operators to handle the workflow timely. Dedicated personal to handle anxious patients can be available during certain time slots. Trainees or unexperienced operators may be advised to perform simple examinations.

Optionally, the type of human-machine communication may be an autonomous communication provided between the medical imaging system and the specific patient, and may comprise e.g. verbal communication, such as verbal instructions during preparation or scanning phases, written communication, e.g. by use of a display, user prompt etc., or the like.

In an embodiment, the historical patient-specific clinical workflow data comprises log file data relating to previous medical imaging exams of the specific patient. These data may comprise log files of the medical imaging system or of a similar system, and may be used to identify previous workflow issues of the specific patient, such as a delay, scan abort, unexpected or scan-disruptive events, or the like.

Thus, the patient profile may be generated on the basis of experience.

According to an embodiment, the historical patient-specific clinical workflow data may comprise log file data relating to previous medical imaging exams of a patient determined, by the data processing unit, based on the generated patient profile to have an at least similar patient profile compared to the specific patient.

Optionally, the generated patient profile may be classified, e.g. by use of pattern recognition. For example, a classifier may be used for this purpose, which is a mathematical function, implemented by a classification algorithm that maps input data to a category.

Thus, even if historical data for the specific patient are not yet available, such data from comparable patients may be considered.

In an embodiment, a scan protocol may be at least partly extracted from the log file data, the scan protocol comprising one or more of an image acquisition timing, an order, a polarity, a repetition frequency of RF pulse, and applied magnetic field gradients.

Thus, a suitable scan protocol may be determined at least partly based on historical data. This can be performed autonomously.

According to an embodiment, the method may further comprise:
- obtaining, by the computational prediction model, a current patient state, wherein the current patient state refers to a state in the presence of the patient in the environment of the medical imaging system;
- wherein predicting the patient-specific operation workflow is further based on the obtained current patient state.

For example, the current patient state may be measured by parameters as current blood pressure and heart rate, time elapsed since last meal, e.g. indicating the patient to be sleepy, hungry, restless etc., current medication, e.g. if affecting attentiveness, current mood etc. These data may be obtained by suitable questionnaire performed when patient arrives at the imaging facility, by taking and processing images of the patient, by measurements, etc.

Thus, the workflow may be adapted more precisely.

In an embodiment, predicting the patient-specific operation workflow is further based on staff feedback data comprising one or more of: a staff expert-level, a staff availability, and a preferred type of interaction with the patient and/or the medical imaging system.

The staff feedback data may be obtained e.g. via a input data interface, and may be mapped to the patient and/or the examination procedure. The staff feedback may comprise one or more of an expert level, availability, a preferred type of interaction with patients etc.

According to a second aspect, there is provided a method for training a computational model, the model to be trained for at least partly operating a medical imaging system, the method comprising:
- obtaining, by a training data processing module, a patient profile parametrized based on at least time-invariant patient information and a current patient state,
- obtaining, by the training data processing module, log data assigned to the medical imaging system based on the obtained patient profile to identify log data directly assigned to the specific patient or to identify log data of a patient having at least a similar patient profile,
- generating, by the training data processing module, training data by correlating the obtained patient profile with the obtained log data, the training data having at least one label indicating the correlation between the obtained patient profile with the obtained log data, and
- providing the generated training data to the model.

The method may use one or more suitable learning algorithms, which may particularly suitable for supervised learning. The training data may be labelled to be machine-readable.

According to a third aspect, a medical imaging system is provided. The medical imaging system comprises at least a data processing unit, the data processing unit adapted to compute a model adapted to operate at least parts of the medical imaging system, the processing unit configured to:
- obtain time-invariant patient information,
   generate a patient profile parametrized based on at least the obtained time-invariant patient information,
- obtain at least one current clinical workflow parameter, and
- provide a prediction comprising at least a patient-specific operation workflow based on at least correlating the generated patient profile with the obtained current clinical workflow parameter, the predicted patient-specific operation workflow to be used for operating the medical imaging system.

Optionally, the medical imaging system comprises an imaging device.

According to a fourth aspect, a computer program element for operating a medical imaging system is provided, which, when being executed by a data processing unit, is adapted to perform the method according to the first aspect and/or the second aspect.

According to a fifth aspect, there is provided a computer readable medium having stored the computer program element of the fourth aspect.

These and other aspects of the present invention will become apparent from and elucidated with reference to the embodiments described hereinafter.

### BRIEF DESCRIPTION OF THE DRAWINGS

Exemplary embodiments of the invention will be described in the following drawings.
Fig. 1 illustrates a medical imaging system according to an embodiment of the present invention.
Fig. 2 illustrates in a block diagram predicting and/or providing a patient-specific operation workflow according to an embodiment of the present invention.
Fig. 3 illustrates in a block diagram training of a computational prediction model according to an embodiment of the present invention.
Fig. 4 illustrates in a flow diagram of a method for operating a medical imaging system according to an embodiment of the present invention.

### DETAILED DESCRIPTION OF EMBODIMENTS

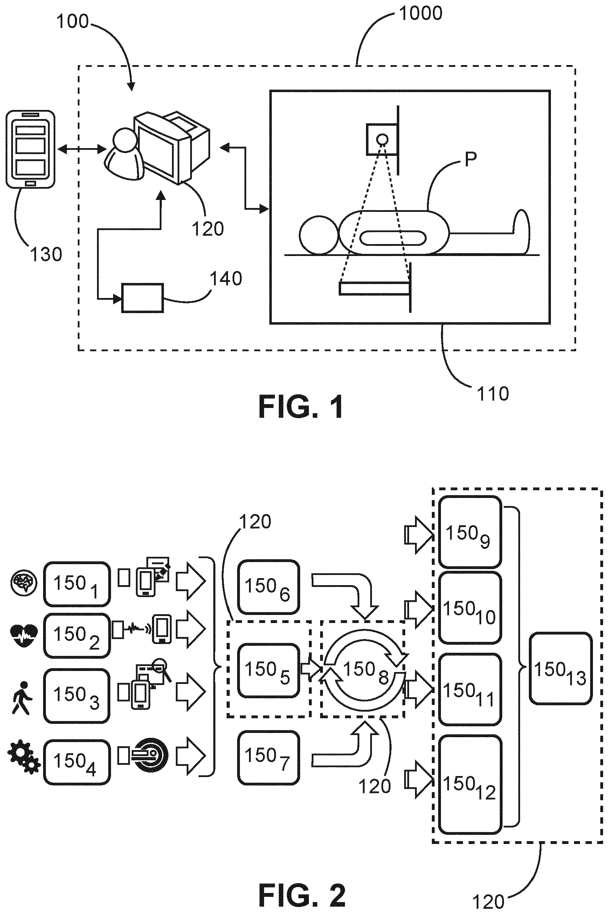

Fig. 1 illustrates a medical imaging system 100, preferably located in a medical imaging facility, wherein medical imaging system 100 may, for example, be adapted to provide magnetic resonance imaging (MRI), computed tomography (CT) imaging, X-ray imaging, or the like, of a patient P to be examined, which is in the following also referred to as the specific patient. For this purpose, medical imaging system 100 comprises a medical imaging device 110, which may be adapted for medical imaging using at least one of the above imaging techniques. Further, medical imaging system 100 comprises a controller 120, which may be a suitable type of computer, adapted to control medical imaging system 100 and/or interrelated systems. In particular, controller 120 may be adapted to process a computational prediction model as described in more detail further below, and to operate medical imaging system 100 based on the processed computational prediction model. Controller 120, which may be referred to as a data processing unit, may comprise one or more of an input data interface, an output data interface, a communication interface to interrelated systems, devices etc., such as a clinical information system, a computer application, processed by e.g. a personal computer to be operated by patient P, a mobile device to be carried and/or operated by patient P, etc. In Fig. 1, a computer adapted to process the above computer application is designated with reference sign 130, wherein computer 130 is, by way of example, provided as a mobile device, wherein the computer application may also be processed on a desktop computer, or the like. Computer 130 may be coupled to medical imaging system 100, and particularly to controller 120, by a communication interface, a communication network, such as the internet, or the like. It is noted that computer 130, in at least some embodiments, may comprise or may interact with one or more sensors (not shown) adapted to measure a heart rate, a blood pressure, a stress level, sleeping behavior, or the like, of patient P. Further, medical imaging system 100 may comprise one or more sensors 140, such as a camera, a heart rate monitor, a blood pressure monitor, or the like, adapted to collect corresponding patient data on site at medical imaging system 100. In at least some embodiments, one or more of sensors 140 may be arranged within a bore of medical imaging device 110 or next to it.

Further, medical imaging system 100 may be embedded in or may interact with a clinical information system, which is designated here with reference sign 1000. Such a clinical information system 1000 may be adapted to, e.g. by use of a database and/or data interface to medical imaging system 100, collect and/or provide patient data, such as medical patient records, insurance data, historical patient-specific clinical workflow data, a health status of a patient, etc. There may be at least one data interface adapted to allow data transfer between medical imaging system 100 and clinical information system 1000.

In addition, medical imaging system 100 may be adapted to be operated at least partly autonomously, wherein three operation modes may be distinguished - fully-autonomous, semi-autonomous, and fully-staff supported. In the fully-autonomous operating mode, one or more of examination scheduling, patient communication, scan protocol selection and/or adaptation, and the imaging procedure may be carried out without human intervention, i.e. staff intervention. In the semi-autonomous operating mode, some parts of the examination may be carried out autonomously without human intervention, while other part of the examination may be carried out by staff. In the fully-staff supported operating mode, at least most relevant or all parts of the examination may carried out by staff. An individual patient-specific operation workflow may be assigned to each of these operating modes.

Fig.2 illustrates in a block diagram predicting and/or providing a patient-specific operation workflow. According to Fig. 2, blocks 150₁, 150₂, 150₃, 150₄ represent input data or the collection of input data for the above computational prediction model, wherein the input data represented by blocks 150₁, 150₂, 150₃, 150₄ may be referred to as time-invariant patient information data. The time-invariant patient information data comprises one or more of at least one psychological patient parameter (see block 150₁), at least one physiological patient parameter (see block 150₂), a patient health status (see block 150₃), and historical patient-specific clinical workflow data (see block 150₄). In a block 150₅, the time-invariant patient information is processed to a parametrized patient profile assigned to patient P, which may be referred to as intermediate output data.

Block 150₅ may be referred to as a processing block, wherein a dashed line surrounding this block indicates that the block forms at least part of the above computational prediction model, which is processed by controller 120.

Further, blocks 150₆, 150₇ represent further input data for the above computational prediction model, which input data is different to the above time-invariant patient information data. In particular, block 150₆ represents current clinical workflow parameter comprising one or more of current delays in each procedure step of examinations carried out in the medical imaging facility, a number of re-scans required in the medical imaging facility, etc. Block 150₇ represents staff feedback data comprising one or more of a staff expert-level, a staff availability, and a preferred type of interaction with the patient and/or medical imaging system 100. Optionally, further input data may be formed by a current patient state, wherein the current patient state refers to a state in the presence of patient P in the environment of medical imaging system 100.

A block 150₈ represents the above computational prediction model or its processing, respectively. Block 150₈ may also be referred to as a processing block wherein a dashed line surrounding this block indicates that the block forms at least part of the above computational prediction model, which is processed by controller 120. Blocks 150₉, 150₁₀, 150₁₁, 150₁₂ represent output data of the above computational prediction model. These output data may also be referred to as a patient-specific operation workflow. In other words, a part or all of these output data may be combined and/or further processed to the patient-specific operation workflow, as indicated by a block 150₁₃.

In more detail, block 150₁ represents psychological parameter of patient P, wherein these psychological patient parameter may comprise one or more of or may be indicative for one or more of a certain behavioral pattern, a proneness to anxiety, a risk for unexpected claustrophobia etc., a type of biorhythm, such as "early bird" vs. "night owl", a character type, such as "fighter", which may be referred to as a first character type, "academic", which may be referred to as a second character type, and "social", which may be referred to as a third character type, etc. As indicated in Fig. 2, these data may, for example, be obtained from patient P prior to the examination or the date of examination, respectively. In particular, these data may be derived from measurements of computer 130 or from the computer application that runs on computer 130, by a questionnaire provided by the computer application or in paper form, etc. Additionally or alternatively, at least a part of the psychological patient parameter may be obtained from the one or more sensors 140 at site of medical imaging system 100.

Block 150₂ represents physiological patient parameter of patient P, wherein these physiological patient parameter may comprise one or more of a heart rate, a blood pressure, breathing pattern, ability to hold breath, etc. As indicated in Fig. 2, these data may, for example, be obtained from patient P prior to the examination or the date of examination, respectively. In particular, these data may be derived from measurements of computer 130 or from the computer application that runs on computer 130, by a questionnaire provided by the computer application or in paper form, etc. Additionally or alternatively, at least a part of the physiological patient parameter may be obtained from the one or more sensors 140 at site of medical imaging system 100.

Block 150₃ represents other patient information and/or condition, such as weight, height, BMI, disabilities, motion impairment, implants carried in the body, allergies, etc. of patient P. As indicated in Fig. 2, these data may, for example, be obtained from patient P prior to the examination or the date of examination, respectively. In particular, these data may be derived from measurements of computer 130 or from the computer application that runs on computer 130, by a questionnaire provided by the computer application or in paper form, etc. Additionally or alternatively, at least a part of the psychological patient parameter may be obtained from the one or more sensors 140 at site of medical imaging system 100. Optionally, at least a part of the other patient information and/or condition of patient P may be obtained from an electronic patient record, insurance information, or the like, collected and/or provided, for example, by clinical information system 1000.

Block 150₄ represents historical patient-specific clinical workflow data of patient P or, alternatively, of another patient having an at least similar patient profile. These data comprises previous log file data relating to previous medical imaging exams of patient P or a patient having a similar patient profile. Further, these data may be indicative for delays caused by or with the patient, or other unexpected events.

Block 150₅ represents a processing block, which may be processed by use of the above computational prediction model or another suitable processing module.

In at least some embodiments, block 150₉ represents a predicted and/or proposed patient engagement regarding patient P, comprising e.g. special preparation of patient P, such as breath hold, delivering dedicated information regarding the examination procedure, addressing anxiety, etc.

In at least some embodiments, block 150₁₀ represents a predicted and/or proposed patient experience which can be understood as how experienced the patient is in this type of examination. These data may comprise predicting and/or proposing of e.g. staff assignment depending on patient P's individual needs, selecting or composing a suitable scan protocol based on an expected level of patient cooperation, etc.

In at least some embodiments, block 150₁₁ represents a predicted and/or proposed staff engagement, such as e.g. staff assignment depending on certain examination procedures according to an individual expert-level and/or training status of the individual staff, staff assignment depending on a desired type of interaction, etc.

In at least some embodiments, block 150₁₂ represents a predicted and/or proposed workflow optimization, such as selecting or composing an imaging scan protocol for certain examinations according to patient P's abilities, such as e.g. free breathing, breath hold, etc., or assignment of qualified staff qualified for e.g. sensor placement, contrast agent delivery, etc.

Block 150₁₃ represents further processing of the above data to the patient-specific operation workflow. The latter may comprise the predicted patient-specific operation workflow comprises one or more of a schedule for examining the patient P, a scan protocol based on which imaging for examining patient P is controlled, the scan protocol comprising at least an image acquisition parameter, a level of staff engagement for examining patient P, the level of staff engagement affecting a level of the autonomous operating mode of medical imaging system 100, a guidance for staff for examining patient P, the guidance comprising one or more of a visualization output, a traffic light system, and an audio output, to be presented to the staff, and a type of human-machine communication between the medical imaging system and patient P, the type of human-machine communication adapted to the patient profile.

In at least some embodiments, one or more of blocks 150₅, 150₈, and 150₁₃ are adapted to determine a risk level of patient P, wherein the risk level is assigned to a specific one of the operational modes of medical imaging system 100. On this basis, medical imaging system 100 may be operated based on the specific of the operational modes that is selected depending from the determined risk level. Further, in at least some embodiments, the operational mode is selected depending on whether the determined patient-specific risk level matches an assigned threshold value or an assigned threshold range.

In the following, application examples of one or more of the above embodiments will be described.

In at least some embodiments, patient P may be scheduled for a brain MRI. Prior to the examination, patient P is reminded of the examination via the above computer application, e.g. by use of a personal computer, a mobile device, or the like. Additionally, patient P is presented a questionnaire to be filled upfront. For example, a question may be: "do you have metal implants? Are you anxious in small and narrow rooms?" etc. Optionally, other patient information, such as weight, allergies etc. may be asked. The answers may be sent to the medical imaging facility for further processing. For example, in a database of the medical imaging facility, patient P is already present and known from an initial MRI exam in the past. At that time, for example, patient P presented anxious and had problems to stay in medical imaging device 110 for more than 20 minutes. The above computational prediction model may be adapted to correlate such information, e.g. via a neural network, and predicts and/or proposes several options for the scheduling of patient P. For example, the scan protocol may be chosen and/or adapted in such a way that only fast imaging sequences are applied and the whole exam does not exceed the 20 minutes. Moreover, the staff may be advised to spend more time in talking to patient P in order to calm him down. Thus, the time slot for this patient may be extended or planned at the end of the work day.

In at least some embodiments, patient P may be scanned for a cardiac MR exam. Such exams critically depend on the heart rate of the patient. A lower heart rate may result in better image quality and a lower probability of scan interruption. Overall scan time also depends on the heart rate and its variability in a non-linear way. Cardiac scans also benefit from breath holds by the patient, improving image quality and reducing scan time. It is known that the maximal duration of breath holds (MDB) depends on the current physiological and psychological state of the patient. A high heart rate indicates a high metabolic rate which increases oxygen consumption and lowers MDB. Similarly, a meal that was just taken may result in considerably shorter MDB because lung volume is decreased and metabolic rate increased due to digestion. Any psychological deviation form a calm state shortens MDB and increases HR. Above and further relevant input parameters, such as hear rate, breathing rate, blood pressure in comparison to average blood pressure at home, time elapsed after last meal, moisture of palms of hands indicating nervousness, current medication, further suitable parameters, may indicate the current state of patient P and may be measured when patient P arrives at the medical imaging facility and ideally again at the beginning of the cardiac exam. The computational prediction model may use a correlation of these parameters to critical intermediate quantities as MDB to predict those. Alternatively, the computational prediction model may directly propose certain MR protocols and their variants to adapt to the particular current MDB and HR. Scheduling may be changed as well on the fly if a patient is still particularly nervous, giving him some time and appropriate help to calm down. Overall scan time may be predicted to optimize scheduling for patient P and other patients.

In at least some embodiments, patient P may be scheduled for a brain examination with contrast. The patient has never been scanned before. However, based on the psychological parameters acquired through the above computer application and/or the type of examination scheduled, the computational prediction model predicts that patient P will most likely become afraid of the situation and may refuse to be examined when shown the MRI scanner, i.e. medical imaging device 110. The computational prediction model proposes that technologist A should handle this patient, because from historical data and/or staff feedback data it is known that technologist A has often been successful in handling patients of this type and in communicating in such a way that the patient feels less anxious.

In at least some embodiments, patient P may be scheduled for a spin echo MRI. MRI protocols are the combination of various MRI sequences which can optimally asses a particular region of the body. There may be a protocol pool to choose for a particular type of scan. Spin echo sequence may require one or more of adapting the field of view, the number of slices and sequence of operations with 90 and 180 degree RF pulse, which are captured from the log file. There may be different possibilities to generate the sequence of certain contrast even with pre-defined protocols. The log files may be extracted to identify timing, order, polarity and repetition frequency of RF pulse and applied magnetic field gradients. The extracted log information from similar patients, which may be identified by classification using the computational prediction model, of one particular scan can identify previous workflow issues and customization of the sequence of operations there by protocols.

In at least some embodiments, in one of the autonomous imaging operation modes of medical imaging system 100, an autonomous communication with patient P may be needed. Such a communication, which may comprise written and/or verbal communication, the style of communication may be adapted to the patient profile of patient P, by the computational prediction model. For example, a patient P having the profile of a Fighter probably prefers a rather fast and efficient action and respond best to instructions delivered in a short and compact manner. Specifically, the best written communication style will be a set of bullet points and verbal style a series of orders which just have to be followed. A patient P having an academic profile probably prefers having a craving to understand why they are being asked to follow an instruction. As such, a preferred written or verbal communication style will be an explanation of why the instruction is given attached to the instruction itself. A patient P having a social profile probably has a craving to feel connected to other patients in a similar situation. As such, a preferred written or verbal communication style may be an illustration of how somebody similar to them has already undergone the procedure before the instruction is given.

In at least some embodiments, different patient profiles will guide staff to approach patient P with a certain behavior, e.g. patients who are very knowledgeable may require staff willing and able to provide a lot of clinical and technical background. Anxious patients, for example, may require staff who are willing to take care of and calm down the patient, and who are motivated by patient satisfaction. According to the generated patient profile, the staff can be informed before seeing the patient on how to approach the patient best. For some patients, dedicated staff members may even be selected to approach and talk to the patient. Optionally, this can be indicated in the scheduling board and presented to the staff, e.g. with a traffic light system, wherein green color may be assigned to cases where no dedicated staff is required, yellow may be assigned to cases where patient P requires much information about the examination, and red color may be assigned to an anxious patient that requires extra care by the staff.

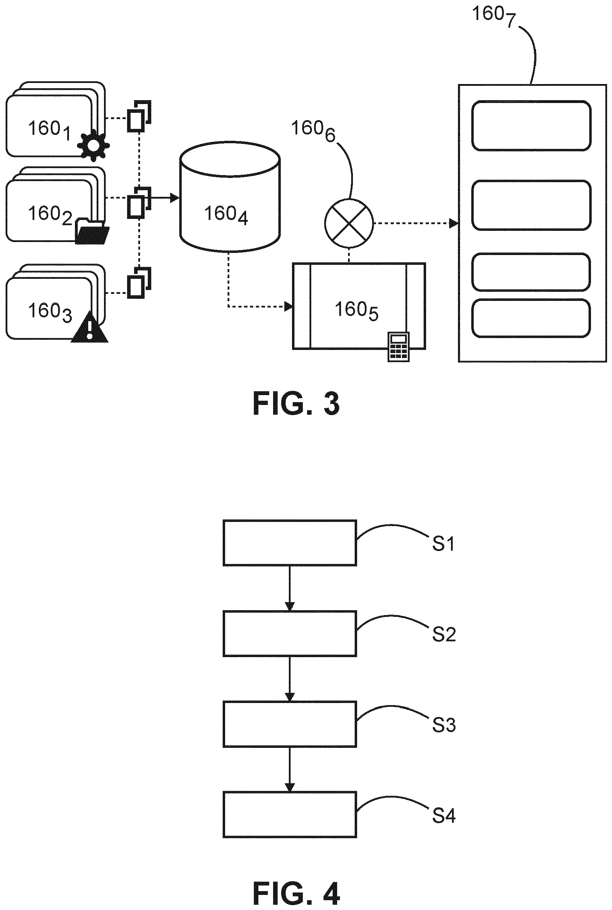

Fig. 3 illustrates in a block diagram a method for training the above computational prediction model that is to be trained for at least partly operating medical imaging system 100. Blocks 160₁, 160₂, 160₃ represent input data and/or data sources and may comprise one or more patient metrics, log files of medical imaging system 100, and historical clinical workflow data. These input data may be collected in a central repository 160₄, which may comprise e.g. a database. The computational prediction model, which in Fig. 3 is represented by block 160₅, accesses or obtains the data from central repository 160₄. In block 160₆, output data of the computational prediction model are applied to medical imaging system 100, preferably in real-time, wherein block 160₇ represents the above patient-specific operation workflow. In particular, the method comprises obtaining, by a training data processing module, a patient profile parametrized based on at least time-invariant patient information and a current patient state, obtaining, by the training data processing module, log data assigned to the medical imaging system based on the obtained patient profile to identify log data directly assigned to the specific patient or to identify log data of a patient having at least a similar patient profile, generating, by the training data processing module, training data by correlating the obtained patient profile with the obtained log data, the training data having at least one label indicating the correlation between the obtained patient profile with the obtained log data, and providing the generated training data to the model. In other words, there is provided a method for training a computational model, the model to be trained for at least partly operating a medical imaging system. The method comprises obtaining, by a training data processing module, a patient profile parametrized based on at least time-invariant patient information and a current patient state. The method further comprises obtaining, by the training data processing module, log data assigned to the medical imaging system based on the obtained patient profile to identify log data directly assigned to the specific patient or to identify log data of a patient having at least a similar patient profile. Further, the method comprises generating, by the training data processing module, training data by correlating the obtained patient profile with the obtained log data, the training data having at least one label indicating the correlation between the obtained patient profile with the obtained log data, and providing the generated training data to the computational prediction model.

Fig. 4 illustrates in a flow diagram a method for operating medical imaging system 100. The method is at least partly carried out by a data processing unit, which here is the controller 120. In a step S1, the above time-invariant patient information is obtained by the above computational prediction model. In a step S2, the computational prediction model generates the above patient profile parametrized based on at least the obtained time-invariant patient information. In a step S3, the computational prediction model obtains at least one current clinical workflow parameter. In a step S4, the computational prediction model provides a prediction comprising at least a patient-specific operation workflow based on at least correlating the generated patient profile with the obtained current clinical workflow parameter, the predicted patient-specific operation workflow to be used for operating the medical imaging system.

It should to be noted that embodiments of the invention are described with reference to different subject-matter. In particular, some embodiments are described with reference to method-type claims, whereas other embodiments are described with reference to device-type claims. However, a person skilled in the art will gather from the above, and the following description that, unless otherwise notified, in addition to any combination of features belonging to one type of subject-matter, also other combinations between features relating to different subject-matters is considered to be disclosed with this application.

All features can be combined to provide a synergetic effect that is more than the simple summation of the features.

While the invention has been illustrated and described in detail in the drawings and foregoing description, such illustration and description are to be considered illustrative or exemplary, and not restrictive. The invention is not limited to the disclosed embodiments.

Other variations to the disclosed embodiments can be understood, and effected by those skilled in the art in practicing the claimed invention, from a study of the drawings, the disclosure, and the dependent claims.

In the claims, the word "comprising" does not exclude other elements or steps, and the indefinite article "a" or "an" does not exclude a plurality. A single processor, or other unit, may fulfil the functions of several items recited in the claims. The mere fact that certain measures are recited in mutually different dependent claims does not indicate that a combination of these measures cannot be used to advantage. Any reference signs in the claims should not be construed as limiting the scope.

### LIST OF REFERENCE SIGNS:

- 1000: clinical information system
- 100: medical imaging system
- 110: medical imaging device
- 120: controller
- 130: computer
- 140: sensor
- 150: block (e.g. functional module, software module, etc.)
- 160: block (e.g. functional module, software module, etc.)

## Claims

1. A method for operating a medical imaging system (100), the method carried out by use of a data processing unit (120), the method comprising:
- obtaining (S1), by a computational prediction model, time-invariant patient information,
- generating (S2), by the computational prediction model, a patient profile parametrized based on at least the obtained time-invariant patient information,
- obtaining (S3), by the computational prediction model, at least one current clinical workflow parameter, and
- providing (S4), by the computational prediction model, a prediction comprising at least a patient-specific operation workflow based on at least correlating the generated patient profile with the obtained current clinical workflow parameter, the predicted patient-specific operation workflow to be used for operating the medical imaging system (100).

2. The method according to claim 1, further comprising:
- determining, by the computational prediction model, a patient-specific risk level assigned to a specific one of a selection of operational modes of the medical imaging system, and
- operating the medical imaging system based on the specific one of the selection of operational modes that is selected depending from the determined risk level.

3. The method according to claim 2, wherein the operational mode is selected depending on whether the determined patient-specific risk level matches an assigned threshold value or an assigned threshold range.

4. The method according to claim 2 or 3, wherein the selection of operational modes comprises a fully-autonomous operation mode, a semi-autonomous operation mode, and a fully-staff supported operation mode.

5. The method according to any one of the preceding claims, wherein the time-invariant patient information comprises one or more of at least one psychological patient parameter, at least one physiological patient parameter, a patient health status, and historical patient-specific clinical workflow data.

6. The method according to any one of the preceding claims, wherein the time-invariant patient information is at least partly remotely obtained in an electronic manner from a computer application to be operated by the specific patient.

7. The method according to any one of the preceding claims, wherein the predicted patient-specific operation workflow comprises one or more of:
- a schedule for examining the specific patient,
- a scan protocol based on which imaging for examining the specific patient is controlled, the scan protocol comprising at least an image acquisition parameter,
- a level of staff engagement for examining the specific patient, the level of staff engagement affects a level of an autonomous operating mode of the medical imaging system,
- a guidance for staff for examining the specific patient, the guidance comprising one or more of a visualization output, a traffic light system, and an audio output, to be presented to the staff, and
- a type of human-machine communication between the medical imaging system and the specific patient, the type of human-machine communication adapted to the patient profile.

8. The method according to any one of the preceding claims, wherein the historical patient-specific clinical workflow data comprises log file data relating to previous medical imaging exams of the specific patient.

9. The method according to any one of claims 1 to 7, wherein the historical patient-specific clinical workflow data comprises log file data relating to previous medical imaging exams of a patient determined, by the data processing unit, based on the generated patient profile to have an at least similar patient profile compared to the specific patient.

10. The method according to claim 8 or 9, wherein, a scan protocol is extracted from the log file data, the scan protocol comprising one or more of an image acquisition timing, an order, a polarity, a repetition frequency of RF pulse, and applied magnetic field gradients.

11. The method according to any one of the preceding claims, further comprising:
- obtaining, by the computational prediction model, a current patient state, wherein the current patient state refers to a state in the presence of the patient in the environment of the medical imaging system,
- wherein predicting the patient-specific operation workflow is further based on the obtained current patient state.

12. The method according to any one of the preceding claims, wherein predicting the patient-specific operation workflow is further based on staff feedback data comprising one or more of: a staff expert-level, a staff availability, and a preferred type of interaction with the patient and/or the medical imaging system.

13. A medical imaging system (100), comprising a data processing unit (120), the data processing unit (120) adapted to compute a prediction model adapted to operate at least parts of the medical imaging system, the processing unit configured to:
- obtain time-invariant patient information,
- generate a patient profile parametrized based on at least the obtained time-invariant patient information,
- obtain at least one current clinical workflow parameter, and
- provide a prediction comprising at least a patient-specific operation workflow based on at least correlating the generated patient profile with the obtained current clinical workflow parameter, the predicted patient-specific operation workflow to be used for operating the medical imaging system.

14. A computer program element for operating a medical imaging system, which, when being executed by a data processing unit, is adapted to perform the method according to any one of claims 1 to 12 or the method according to claim 13.

15. A computer readable medium, having stored the computer program element of claim 14.
